Europäisches Patentamt

European Patent Office

Office européen des brevets

(11) Publication number: **0 470 681 A2**

## EUROPEAN PATENT APPLICATION

(21) Application number: **91250219.2**

(22) Date of filing: **06.08.91**

(51) Int. Cl.5: **C12N 5/00, C12M 3/00, A61L 27/00**

(30) Priority: **08.08.90 JP 211440/90**
**28.02.91 JP 57765/91**

(43) Date of publication of application:
**12.02.92 Bulletin 92/07**

(84) Designated Contracting States:
**DE FR GB IT**

(71) Applicant: **W.R. Grace & Co.-Conn.**
**Grace Plaza, 1114 Ave. of the Americas**
**New York New York 10036(US)**

(72) Inventor: **Takezawa, Toshiaki**
**Nifty 34-302**
**Isehara-shi(JP)**
Inventor: **Kubota, Sunao**
**21-24 Higashi 3-chome**
**Kunitachi-shi, Tokyo-to(JP)**
Inventor: **Mori, Yuichi**
**1642-212 B-4, Kamariva-cho**
**Kanagawa-ken(JP)**
Inventor: **Yamazaki, Manabu**
**Corpo. Tsurumaki A-405, 1200-1 Tsurumaki**
**Hadano-shi, Kanagawa-ken(JP)**

(74) Representative: **UEXKÜLL & STOLBERG**
**Patentanwälte**
**Beselerstrasse 4**
**W-2000 Hamburg 52(DE)**

(54) Cell clusters or sheets comprising a plurality of cell species and preparations thereof.

(57) The present invention provides a cell cluster or sheet comprising a plurality of cell species having a regulated size and a regulated composition of the cell species and a method of preparing the same by using a cell culture substrate comprising a temperature-responsive polymeric compound and a cell adhesive substance.

EP 0 470 681 A2

## Technical Field

The present invention relates to a cell cluster or sheet comprising a plurality of cell species and a method of preparing the same. More specifically, it relates to a cell cluster or sheet comprising a plurality of cell species having a regulated size and a regulated composition of cell species. The cell cluster or sheet is useful for efficient production of cellular products and also useful as a prosthesis for repairing damaged or diseased parts of living tissue or as a simulation system to evaluate the effect of drugs and so on, on a living body.

## Background Art

Organs or tissues are formed from an assembly of different species of cells, and specific functions of organs or tissues are caused and controlled by interactive behaviors of different species of cells. For example, blood vessel is made of tunica intima, tunica media and tunica adventitia. On tunica intima there is a layer of endothelial cells, in tunica media smooth muscle cells exist and in tunica adventitia there are fibroblasts. It is known that various kinds of chemical mediators and hormones are secreted from each cell and control the functions of the other cells. For example, a hormone which targets smooth muscle cell contraction is secreted from endothelial cells. In addition, a growth factor secreted from fibroblasts enhances the proliferation of endothelial cells.

On the other hand, skin is made of epidermis and dermis, and each layer is made of epithelial cells and fibroblasts. There are factors which are secreted from the fibroblasts to control proliferation and differentiation of epithelial cells and factors which are secreted from the epithelial cells to enhance proliferation of fibroblasts. Mutual cell interaction is known to effectively control the functions of skin.

Liver is made up of parenchymal cells, hepatocytes and sinusoidal cells including endothelial cells, stellate cells and so on. These cells are believed to control liver function. Accordingly, a co-culture system using different cell species which form the tissue or organ is considered to be much more adequate for maintaining the tissue or organ specific functions than mono-culture systems made from only one cell species.

The following evidence indicates the performance of co-culture systems. A co-culture system comprising islet cells and fibroblasts significantly increased insulin secretion from the islet cells as compared with a mono-culture system of islet cells alone (Rabionovitch, A. et al., *DIABETES* (1979) 28:1108). A co-culture system comprising hepatocytes and fibroblasts effectively prolonged the viability of the hepatocytes as compared with a mono-culture system of hepatocytes alone (Yoshizato, K., *Abstract of the 4th Meeting for Primary Culture of Hepatocytes* (1988) 11).

Most conventional co-culture systems have been carried out by culturing a mixture of different cell species or sequentially culturing different cell species on culture substrates. Namely, the co-culture systems developed so far are two-dimensional. Two-dimensional culture is effective for cell proliferation processes but inadequate for cell differentiation. Since cell differentiation is necessary for most cells to exhibit *in vivo* specific functions, co-culture of cells on a two-dimensional substrate is not optimal. The problems associated with two-dimensional co-culture systems exist because most *in vivo* cells are not present in a two-dimensional mode but are in a three-dimensional mode. Further, the two-dimensional culture substrate binds and restricts the cells so strongly that the cells find it difficult to regenerate *in vivo* specific structures and to exhibit *in vivo* functions.

An additional problem associated with conventional two-dimensional substrate co-culture is that it is almost impossible to harvest the cultured cells from the substrate and maintain a usable shape, such as a cell sheet or a cell cluster. Difficulties associated with conventional harvesting are due to the use of cell detaching agents, such as trypsin and EDTA. The cell detaching agents destroy cell-to-cell junctions which are necessary to maintain the shape such as a cell sheet and a cell cluster. It is necessary to maintain the cell cluster or sheet shape in order to use the co-cultured cells as a prosthesis to repair damaged or diseased focuses and/or simulate systems of tissues or organs to evaluate biological activity of test substances.

Attempts have been made to prepare a cell sheet or cell cluster without the use of cell detaching agents. Conventional attempts can be classified into two types. The first method makes cell clusters by spontaneously aggregating suspended cells on non-adhesive substrates such as agar and agarose coating (Carlsson, J., et al., *Spheroids in Cancer Research* (1984), 1, edited by Acker, H., et al., Springer-Verlag), and polyhydroxyethyl methacrylate coating (Landry, J., et al., *J. Cell Biol.* (1985) 101:914). The second method makes cell clusters by spontaneously detaching the cells without use of detaching agents from semi-adhesive substrates on to which the cells are weakly attached. Proteoglycans coating (Koide, N., et al., *Biochem. Biophys. Res. Commu.* (1989) 161:385) and positively-charged polystyrene (Koide, N., et al., *Experimental Cell Research* (1990) 186:227) have been used as as such semi-adhesive substrates. These two methods have been applied to the co-culture systems of a plurality of cell species.

A cell cluster composed of HeLa cells and fibroblasts was prepared by the first method using an agar coated substrate (Sasaki, T., et al., *Cancer Research* (1984) 44:345). A cell cluster composed of hepatocytes and endothelial cells was prepared by the second method using a proteoglycan coated substrate (Matsushima, K., et al., *Jpn. J. Artif. Organs* (1990) 19:848).

The major problems associated with the two conventional methods for preparing cell clusters or sheets are the following.

(1) The conventional methods are not applicable to all cell species. The cell clusters or sheets formed by these methods are strongly dependent upon the aggregability and detachability of each cell species, respectively, and the aggregability and detachability are significantly different depending on the cell species.

(2) Using the conventional methods, it is almost impossible to regulate the number of cells constituting the cell clusters or sheets which controls the size of the cell clusters or sheets. Since spontaneous aggregation or detachment of the cells which form the cell clusters or sheets occurs accidentally and unexpectedly, the size of the cell clusters or sheets cannot be regulated and accordingly show a very broad size distribution.

(3) When the cell clusters or sheets are composed of a plurality of cell species by the conventional methods it is difficult to regulate the population number of each cell species constituting the clusters or sheets.

The above described problems are crucial if the obtained cell clusters or sheets are used as an alternative to tissues or organs in order to repair damaged or diseased focuses or to evaluate the biological activity of test substances.

The present invention provides a cell cluster or sheet comprising a plurality of cell species having a regulated size and a regulated composition of the cell species, and a method for preparing a cell cluster or sheet comprising a plurality of cell species without the above described problems.

## Definition

The term "LCST" is used herein to mean a lower critical solution temperature which is a transition temperature of a temperature-responsive polymeric compound between hydration and dehydration.

## Summary of the Invention

A cell cluster or sheet in accordance with the present invention comprises a plurality of cell species and has a regulated size and a regulated composition of the cell species.

Another aspect of the present invention is a method for preparing cell clusters or sheets comprising a plurality of cell species comprises the steps of:

(i) seeding and culturing a plurality of cell species on a cell culture substrate comprising

(a) a temperature-responsive polymeric compound having an LCST lower than the culture temperature and

(b) a cell adhesive substance at a temperature higher than said LCST,

(ii) detaching an assembly of said cell species proliferated on said cell culture substrate from said cell culture substrate by lowering said temperature to a temperature below said LCST, and

(iii) culturing said detached cell assembly in suspension on a non-adhesive substrate to form a cell cluster or sheet.

## Detailed Description of the Invention

According to the present invention, a cell cluster or sheet comprising a plurality of cell species can be obtained. The total cell number of the cluster or sheet and also the population number of each cell species of the cluster or sheet can be precisely controlled. The cell cluster or sheet can be obtained by using a cell culture substrate comprising a temperature-responsive polymeric compound having an LCST lower than the cell culture temperature and a cell adhesive substance capable of effectively allowing the cells to attach and proliferate.

The temperature-responsive polymeric compound solidifies above the LCST and instantly dissolves in an aqueous solution below the LCST. According to the above described change of the temperature-responsive polymeric compound it becomes possible to detach the cells cultured on the substrate the present invention by lowering the temperature to below the LCST. However, most of the cells cannot attach and proliferate on the substrate made from the temperature-responsive polymeric compound alone. Therefore, the cell adhesive substance of this invention is essential. The important feature of this invention is that if the cells form an assembly on the substrate, the assembly does not disintegrate into individual cells and does not lose the cell number by the above described detaching process.

Thus, the method for preparing the cell cluster or sheet of the present invention comprises the steps of:

(i) seeding and culturing a plurality of cell species on the above described cell culture substrate at a temperature higher than the LCST,

(ii) detaching an assembly of a plurality of cell species proliferated from the substrate by lowering the temperature to a temperature lower than the LCST, and

(iii) culturing the detached cell assembly on a non-adhesive substrate to form a cell cluster or sheet.

In step (i), it is possible to seed and culture a plurality of cell species simultaneously or sequentially. The separate or sequential seeding and culturing of a plurality of cell species is preferred. Sequential seeding and culturing is preferred because the attachability and proliferation ability on the substrate are so strongly dependent upon the cell species and the total cell number and the number of each cell species in the cultured cell assembly are not as easy to control by the initial feeding concentration of a plurality of cell species particularly in the simultaneous seeding and culturing. The most preferable method is to seed and culture the second cell species after the first cell species has been seeded and cultured to a confluent stage. Numerous layers of cell species can be applied using the sequential seeding and culturing method. Any practical number of layers is contemplated by this invention, but preferably about ten layers, and most preferably about 2-5 layers. The total cell number and also the number of each cell species of the finally obtained cell cluster or sheet can be easily and precisely controlled by the sequential application method. The number of the confluent first cells on the substrate can be easily and precisely counted before seeding the second cell species using a phase-contrast microscope or other known means. The number of the second cell species seeded and cultured subsequently can be calculated by various methods. The calculation methods include directly counting the number of second cells attached on the confluent first cells under a phase-contrast microscope or measuring the number of unattached second cell species floating in the culture medium and subtracting this number from the initial feeding number of the second cell species.

In addition, in the simultaneous seeding and culturing all of the cell species directly attach to the substrate, while in the separate and sequential seeding and culturing only one species directly attaches to the substrate. Since detachability from the substrate can also be strongly dependent upon the cell species, the latter method is preferred to the former method. Furthermore, the confluent first cells fully wrap the second cells up in a kerchief-like structure during the detaching step, which effectively prevents the loss of the cells from the cell assembly.

In step (iii), the cell assembly detached in step (ii) is transformed into a cell cluster or sheet by suspension culture on a cell non-adhesive substrate. One of the preferred non-adhesive substrates is a temperature-responsive polymer compound coating.

The cell species which can be employed in the present invention include fibroblasts, hepatocytes, endothelial cells, islet cells, keratinocytes, bone cells, nerve cells, muscle cells, kidney cells, brain cells, etc. but in the present invention the cell species are not limited to these species.

Examples of suitable temperature-responsive polymeric compounds having an LCST lower than the culture temperature which can be used in the present invention are poly-N-substituted (meth)-acrylamide derivatives and their copolymers, poly-methylvinylether, polyethylene oxide, etherized methylcellulose, and partially acetylated polyvinyl alcohol. Of these preferred compounds, more preferred are poly-N-substituted acrylamide derivatives, poly-N-substituted methacrylamide derivatives and their copolymers.

Preferred examples of such temperature-responsive polymeric compounds in the present invention are listed below, but this invention is not limited to these examples. The LCSTs of these polymers rise with the sequence of polymers listed below.

Poly-N-acryloyl piperidine, poly-N-n-propyl methacrylamide, poly-N-isopropyl acrylamide, poly-N, N-diethyl acrylamide, poly-N-isopropyl-methacrylamide, poly-N-cyclopropyl acrylamide, poly-N-ethyl acrylamide.

The above described polymers may be homopolymers or copolymers with other monomers. Any hydrophilic monomers or hydrophobic monomers can be used as the monomers for copolymerization. Generally speaking, copolymerization with a hydrophilic monomer will raise the LCST, and copolymerization with a hydrophobic monomer will lower the LCST. With a proper selection of monomers, a copolymer with a desired LCST can be achieved.

Examples of suitable hydrophilic monomers are N-vinylpyrrolidone, vinylpyridine, acrylamide, methacrylamide, N-methyl acrylamide, hydroxyethyl methacrylate, hydroxyethyl acrylate, hydroxymethyl methacrylate, hydroxymethyl acrylate, acrylic acid, methacrylic acid, vinyl sulfonic acid, styrylsulfonic acid their salts and N,N-dimethylaminoethyl methacrylate, N,N-diethylaminoethyl methacrylate, N,N-dimethylaminopropyl acrylamide and their salts, but the present invention is not limited to these compounds.

Examples of suitable hydrophobic monomers are acrylate derivatives and methacrylate derivatives such as ethyl acrylate, methyl methacrylate and glycidyl methacrylate; N-substituted alkyl (meth)acrylamide derivatives such as N-n-butyl (meth)acrylamide;vinyl chloride, acrylonitrile, styrene and vinyl acetate, but the present invention is not limited to these compounds.

The molecular weight of the temperature-responsive polymeric compound which can be employed in the present invention is preferably at least about $1.0 \times 10^5$ and more preferably higher than about $1.0 \times 10^6$. The molecular weight herein means a number average molecular weight obtained form the viscosity. For example, the relationship between the number average molecular weight ($\overline{M}n$) of poly-N-isopropyl acrylamide and its intrinsic viscosity [$\eta$] can be represented by the following equation [Ito, S. and Geromino, R. T., *Sen'i Kobushi Zairyo Kenkyusho Hokoku* (1988) 159:23];

$$[\eta] = 9.59 \times 10^{-5} \; \overline{M}n^{0.65}$$

(in tetrahydrofuran solution at 27°C).

Examples of the cell adhesive substances which can be employed in the present invention are extracellular matrix components, gelatin, lectins, anchorage oligopeptides which are the binding sites of anchorage proteins such as fibronectin, adhesive proteins isolated from shellfish and positively charged polymers. The extracellular matrix components are the substances existing among cells within a living body and include collagen, fibronectin, laminin, vitronectin, proteoglycan, glycosaminoglycan and thrombospondin.

Examples of such positively charged polymers include polylysin, polyhistidine, protamine sulfate, polydimethylaminoethyl acrylate or methacrylate, polydiethylaminoethyl acrylate or methacrylate, polydimethylaminoprophyl acrylate or methacrylate, polyethyleneimine and polyvinylpyridine.

The cell culture substrate which can be used in the method of the present invention typically comprises a coating of a temperature-responsive polymeric compound having an LCST lower than the culture temperature and a cell adhesive substance formed on a supporting material.

Such a substrate is prepared by coating an aqueous solution of a mixture of the temperature-responsive polymeric compound and the cell adhesive substance on all or part of the surface of the supporting material at a temperature below the LCST and drying the coating thus obtained. The substrate can also be prepared by dipping the supporting material into an aqueous mixture solution of the temperature-responsive polymeric compound and the cell adhesive substance at a temperature below the LCST and drying the coating thus obtained.

Further, the substrate used in the present invention can be prepared by forming a layer of the temperature-responsive polymeric compound on a supporting material and then forming a layer of the cell adhesive substance on the layer of the temperature-responsive polymeric compound. Also, the substrate used in the present invention can be prepared by forming a layer of the cell adhesive substance on a supporting material and then forming a layer of the temperature-responsive polymeric compound on the layer of the cell adhesive substance.

The mixing weight ratio of the temperature-responsive polymeric compound to the cell adhesive substance which can be employed in the present invention is typically from about 1:0.01 to about 1:3. This ratio varies depending on the type of the cell adhesive substance used and the cell species used.

The thickness of the coating after it is dried is at least about 0.2 $\mu$m, preferably at least 0.5 $\mu$m and more preferably at least about 1.0 $\mu$m. When the thickness is below about 0.2 $\mu$m, the cell detachability remarkably worsens and it takes a very long time for the detachment of the cells and as a result, the cell functions are rendered unstable.

The supporting material which can be employed in the present invention is preferably transparent or translucent, and is preferably of glass or of plastic. Exemplary plastics include polystyrene, polycarbonate, polymethyl methacrylate, polypropylene, polyethylene, polyester, polyamide, polyvinylidene fluoride, polyoxymethylene, polyvinylchloride, polyacrylonitrile, polytetrafluoro-ethylene and polydimethylsiloxane.

There is no particular limitation on the shape of the supporting material, and it can take various shapes such as a dish, plate, film or sheet.

The examples which follow are given for illustrative purposes and are not meant to limit the invention described herein. The following abbreviations have been used throughout in describing the invention.

| | |
|---|---|
| LCST | - lower critical solution temperature |
| NIPAAm | - N-isopropyl acrylamide |
| PNIPAAm | - poly-N-isopropyl acrylamide |
| PBS | - phosphate buffered saline |
| DMEM | - Dulbecco's modified Eagle's medium |
| FBS | - fetal bovine serum |
| HEPES | - N-2-hydroxyethylpiperadine-N'-2-ethanesulfonic acid |
| HE | - hematoxylin-eosin |
| ABC | - avidin-biotin-peroxidase complex |
| DAB | - 3,3'-diaminobenzidine tetrahydro chloride |

Example 1

N-isopropyl acrylamide monomer (herein "NIPAAm", a product of Eastman Kodak Co.) 50 g was dissolved in benzene 500 ml, and 2,2'-azobisisobutyronitrile 0.2 g was used as the polymerization initiator to conduct polymerization at 60°C for 12 hours in a stream of nitrogen gas with constant agitation. The polymer precipitated in benzene, was decanted, and the precipitate was dissolved in tetrahydrofuran and then purified by precipitation with ethyl ether to obtain poly-N-isopropyl acrylamide (herein "PNIPAAm"). The PNIPAAm thus prepared had a number average molecular weight of $2.0 \times 10^6$, and the LCST of the 0.5% (w/v) aqueous PNIPAAm solution and the LCST of the 1.0% (w/v) PNIPAAm phosphate buffer solution (herein "PBS") were determined by turbidimetry, and they were about 32°C and about 29°C, respectively. Using the 0.5% (w/v) aqueous type I collagen solution derived from pepsinized calf skin (sterilized, a product of Kohken K.K.) a mixed solution containing 0.25% (w/v) PNIPAAm and 0.25% (w/v) collagen as the final concentration was prepared. Using two types of templates having holes of about 9 mm and 15 mm in diameter, the above described mixed solution was aseptically coated onto a commercial hydrophobic dish having a diameter of 60 mm (Falcon) and the coated culture substrates (Type I substrate) having a surface area of about 0.6 and 1.8 $cm^2$, respectively were prepared. The weight mixing ratio of collagen to PNIPAAm in the coating was 1:1 and the coating thickness was about 2 $\mu$m. In addition, Type II substrate was prepared by merely coating the solution containing 0.5% (w/v) PNIPAAm on a commercial tissue culture dish having a diameter of 35 mm (Falcon) at the thickness of about 2 $\mu$m.

Human dermal fibroblasts were obtained and maintained according to the reported method (Yoshizato, K., et al., *Biochem. Biophys. Acta* - (1980) 627:23). Primary parenchymal hepatocytes were isolated from 5 week-old male Sprague-Dawley rats weighing about 150 g (Charles River Japan, Inc.) by the perfusion method of the liver in situ with 0.05% (w/v) collagenase. Pre-warmed (37°C) human dermal fibroblast suspension in the culture medium [Dulbecco's modified Eagle's medium (herein "DMEM") containing 10% (w/v) fetal bovine serum (herein "FBS"), 20mM N-2-hydroxyethylpiperadine-N'-2-ethanesulfonic acid (herein "HEPES"), 100 units/ml penicillin and 100 mg/ml streptomycin] was seeded on the pre-warmed (37°C) above described Type I substrates of 0.6 and 1.8 $cm^2$ in surface area at the initial cell density of about $4.0 \times 10^4/cm^2$, respectively. After 3 days of culturing at 37°C in a humidified atmosphere of 5% by volume of $CO_2$ and 95% by volume of air, the fibroblasts proliferated to a confluent stage. Here, the confirmation of the confluent state and the measurement of the confluent fibroblast density were carried out by an inverted phase-contrast microscopic study. Then the pre-warmed (37°C) above described rat primary parenchymal hepatocyte suspension in the culture medium was seeded on each of the above-described confluent fibroblast monolayers at cell densities of about $1.0 \times 10^4$ and $5.0 \times 10^4/cm^2$, respectively. After 60 minutes of co-culturing, it was confirmed by an inverted phase-contrast microscope that more than 90% of the seeded hepatocytes attached to the fibroblast monolayer. The culture dishes were brought out from the 37°C-incubator to an ambient temperature (about 25°C) and left to stand for about 5 minutes. By this procedure, the hepatocyte-attached fibroblast monolayer was completely detached from the Type I substrate as a cell assembly. The detached cell assembly was rinsed twice with chilled PBS and once with chilled culture medium in order to avoid contamination of PINPAAm and collagen dissolved in the culture medium, and then transferred into pre-warmed (37°) culture medium on the above described Type II substrate. The cell cluster formation and culture were carried out at 37°C in a humidified atmosphere of 5% by volume of $CO_2$ and 95% by volume of air on the Type II substrate and the culture medium was changed every three days.

The number of fibroblasts and hepatocytes in the cell clusters was measured by the following methods. The 4-day cultured cell clusters were fixed in 10% formalin neutral buffer solution for 60 minutes at 4°C. They were dehydrated and embedded in paraffin wax. Sections were cut in the vicinity of its center at a thickness of 2 $\mu$m. After removal of the wax, hematoxylin-eosin (herein "HE") staining by the standard procedure was carried out. For the immunoperoxidase staining, dewaxed sections were immersed in methanol containing 0.3% by weight of $H_2O_2$ for 30 minutes to remove endogenous peroxidase activity. Then the sections were incubated with goat serum for 60 minutes, with rabbit IgG fraction against rat albumin (Cappel, Organon Teknika Corporation) (1:500 dilution in PBS) for 30 minutes, with biotinylated goat antibody against rabbit IgG for 30 minutes, and with avidin-biotin-peroxidase complex (ABC) (Vector Laboratories, Inc.) for 30 minutes in a moist chamber at 37°C. Bound peroxidase was detected by incubation with 0.5 mg/ml DAB (3,3'-diaminobenzidine tetrahydrochloride) (Dojindo Laboratories) containing 0.01% by weight of $H_2O_2$ for 2 minutes. The sections were counterstained in hematoxylin and dehydrated before mounting.

The number of fibroblasts and hepatocytes was calculated from the micrographs of the sections stained with HE and indirect immunoperoxidase as described above, respectively. In addition, the diameter of all the clusters was measured from the phase-contrast micrographs.

The number of hepatocytes and fibroblasts in all the clusters and the size of the cell clusters are shown in in Table I.

TABLE I

Cell Number and Size of Cell Clusters Composed of Fibroblasts and Hepatocytes

| Run No. | Surface Area of Type I Substrate (cm²) | Seeding Density of Hepatocyte [x 10⁴ (cells/cm²)] | Number of Cells | | Diameter of Cell Cluster (μm) |
| --- | --- | --- | --- | --- | --- |
| | | | Fibroblast (cells/section) | Hepatocyte (cells/section) | |
| 1 | 0.6 | 1.0 | 278 ± 51 | 47 ± 20 | 419 ± 6 |
| 2 | 0.6 | 5.0 | 203 ± 36 | 210 ± 36 | 570 ± 22 |
| 3 | 1.8 | 1.0 | 816 ± 86 | 155 ± 36 | 934 ± 34 |
| 4 | 1.8 | 5.0 | 890 ± 48 | 897 ± 225 | 1160 ± 35 |

Cell Number and Diameter were average values in four experiments.

Table I shows that the number of fibroblasts and hepatocytes in the cell clusters can easily and precisely be controlled by the surface area of Type I substrate and the seeding density of hepatocytes, respectively and also that the diameter of the cell clusters is precisely regulated by the total cell numbers in the method of this invention.

Example 2

Pancreatic islets were isolated from WKA adult rats weighing about 300 g by the method of Lacy (Lacy, P. E., et al., *DIABETES* (1967) 16:35). The diameter of the obtained islets ranged from 200 to 500 $\mu$m.

Type I substrate was prepared by coating the same mixed solution containing 0.25% (w/v) PNIPAAm and 0.25% (w/v) collagen as used in Example 1 onto the commercial tissue culture dish having a diameter of 35 mm (Falcon). The weight mixing ratio of collagen to PNIPAAm in the coating was 1:1 and the coating thickness was about 2 $\mu$m. Also Type II substrate was prepared by the same method as in Example 1.

The pre-warmed (37°C) human dermal fibroblast suspension in the same culture medium as in Example 1 was seeded on the pre-warmed (37°C) above described Type I substrate at an initial-cell density of about $4.0 \times 10^4/cm^2$. After 4 days of culturing at 37°C in a humidified atmosphere of 5% by volume of $CO_2$ and 95% by volume of air, the fibroblasts proliferated to a confluent stage. Here, the confirmation of the confluent state and the measurement of the confluent fibroblast density were carried out by an inverted phase-contrast microscopic study. Then, a few pieces of the above described islets suspended in the pre-warmed (37°C) DMEM were seeded on the above described fibroblast monolayer. After 10 hours, it was confirmed by an inverted phase-contrast microscope that the seeded islets fully attached onto the fibroblast monolayer. Then, Type I substrate was brought out from the 37°C-incubator to an ambient temperature (about 25°C). It was confirmed by an inverted phase-contrast microscope that by this procedure the islet-attached fibroblast monolayer gradually detached from the Type I substrate and the detached monolayer aggregated wrapping up the islets on it. In this detaching process, no dissociation of the islets from the fibroblast monolayer was recognized. The cell assembly made from fibroblasts and islets thus obtained was washed 2 to 3 times with PBS and then transferred into the pre-warmed culture medium on the above described Type II substrate and cultured in suspension changing the culture medium every 4 days. This culture process enabled the cell assembly to transfer to a complete cell cluster. After 14 days of culturing on the Type II substrate, the cell cluster was fixed in 10% formalin neutral buffer solution, dehydrated and embedded in paraffin wax. Sections were cut in the vicinity of its center at a thickness of about 2 $\mu$m. After removal of the wax, the HE staining by the standard procedure was carried out. The staining clearly showed that the seeded islets were completely encapsulated with the fibroblasts and the cell cluster obtained was composed of the islets and the fibroblasts.

Example 3

Using the same method as in Example 2, the cell cluster composed of one islet having a diameter of about 250 $\mu$m and human dermal fibroblasts was prepared. The cell cluster was cultured in DMEM containing 10% (w/v) FBS at 37°C in a humidified atmosphere of 5% by volume of $CO_2$ and 95% by volume of air on the Type II substrate by changing the culture medium every 4 days. The amount of insulin secreted from the cell cluster into the culture medium was measured by the standard immunoassay. Even after 40 days of culturing, the amount of insulin secretion, about 33 $\mu$U/islet/day was observed. In addition, after 2 months of culturing, the cell cluster was observed by the above described HE staining and as a result, no necrosis was seen in the islet encapsulated with the fibroblasts. As a control, only an islet of about 350 $\mu$m in diameter was cultured on the Type II substrate under the same conditions as described above without the fibroblasts. After 10 days of culturing, the secretion rate of insulin reduced to about 4$\mu$U/islet/day and the HE staining demonstrated the necrotized inside of the islet after 2 weeks of culturing. These findings suggest that the formation of the cell cluster with the fibroblasts effectively maintains the islet viability and function for a long period of time.

Example 4

Islet cells were prepared from the same islets as in Example 2 by the reported method (Ono, J., et al., *Endocrinol. Japan.* (1977) 24:265). Instead of the islets, the cell cluster of the islet cells and the fibroblasts was prepared by the same method as in Example 2. As the result of the HE staining, it was found that the islet cells were fully encapsulated with the fibroblasts.

Example 5

The pre-warmed (37°C) human dermal fibroblast suspension in the same culture medium as used in Example 1 was seeded on the pre-warmed (37°C) Type I substrate as used in Example 1 at an initial cell density of about 4.0 x $10^4$/cm$^2$. After 3 days of culturing at 37°C in a humidified atmosphere of 5% by volume of $CO_2$ and 95% by volume of air, the fibroblasts proliferated up to a confluent stage in a monolayer. Then a pre-warmed (37°C) human umbilical vein endothelial cells (Sanko Junyaku Co., Ltd.) suspension in the above described culture medium was seeded on the above described fibroblast monolayer at a cell density of about 5.0 x $10^4$/cm$^2$. After 60 minutes, most of the seeded endothelial cells attached to the fibroblast monolayer. Then, the Type I substrate was brought out from the 37°C incubator to an ambient temperature (about 25°C). It was confirmed by an inverted phase-contrast microscope that by this procedure the endothelial cells-attached fibroblast monolayer was gradually detached from the Type I substrate and the detached monolayer aggregated wrapping up the endothelial cells on it. In this detaching process, significant dissociation of the endothelial cells and fibroblasts was not observed.

The cell assembly made from fibroblasts and endothelial cells thus obtained was washed 2 to 3 times with PBS and then transferred into the pre-warmed culture medium on the above described Type II substrate and cultured in suspension changing the culture medium every 4 days. This culture process enabled the cell assembly to transfer to a complete cell cluster. After 14 days of culturing on the Type II substrate, the cell cluster was fixed in 10% formalin neutral buffer solution, dehydrated and embedded in paraffin wax. Sections were cut in the vicinity of its center at a thickness of about 2 $\mu$m. After the wax was removed, the HE staining by the standard procedure was carried out. The staining clearly showed that the seeded endothelial cells were completely encapsulated with the fibroblasts and the obtained cell cluster was composed of endothelial cells and fibroblasts.

**Claims**

1. A cell cluster or sheet comprising a plurality of cell species having a controlled size and a controlled composition of said cell species.

2. A cell cluster or sheet of Claim 1, wherein said cell species are selected from the group consisting of fibroblasts, hepatocytes, endothelial cells, islet cells, keratinocytes, bone cells, nerve cells, muscle cells, kidney cells and brain cells.

3. The cell cluster or sheet of Claim 2, wherein said cell species are fibroblasts and at least one member selected from the group consisting of heptatocytes endothelial cells, islet cells, keratinocytes, bone cells, nerve cells, muscle cells, kidney cells and brain cells.

4. A method for preparing a cell cluster or sheet comprising a plurality of cell species which comprises the steps of:

(i) seeding and culturing a pluralitiy of cell species on a cell culture substrate comprising

(a) a temperature-responsive polymeric compound having an LCST lower than the culture temperature and

(b) a cell adhesive substance, at a temperature higher than said LCST,

(ii) detaching an assembly of a plurality of cell species proliferated on said cell culture substrate from said cell culture substrate by lowering said temperature to a temperature below said LCST, and

(iii) culturing said detached cell assembly on a non-adhesive substrate to form a cluster or sheet.

5. The method of Claim 4, wherein step (i) comprises simultaneously seeding and culturing a plurality of cell species.

6. The method of Claim 4, wherein step (i) comprises seeding and culturing one cell species until it completely covers the cell culture substrate, and then seeding and anchoring at least one other cell species.

7. The method of Claim 4, wherein step (i) comprises

(a) seeding and culturing one cell species until it completely covers the cell culture substrate to form a monolayer of said cell species, and

(b) seeding and culturing another cell species on said monolayer until it completely covers said monolayer and alternately repeating steps (a) and (b) for a predetermined number of times.

8. The method of Claim 4, wherein the composition of said cell species in the cell cluster or sheet is controlled by regulating the concentrations of each cell species seeded.

9. The method of Claim 4, wherein said cell species are selected from the group consisting of fibroblasts, hepatocytes, endothelial cells, islet cells, keratinocytes, bone cells, nerve cells, muscle cells, kidney cells and brain cells.

10. The method of Claim 4, wherein said cell species are fibroblasts and at least one member selected from the group consisting of hepatocytes, endothelial cells, islet cells, keratinocytes, bone cells, nerve cells, muscle cells, kidney cells and brain cells.

11. The method of Claim 4, wherein said temperature-responsive polymeric compound is selected from the group consisting of a poly-N-substituted acrylamide derivative, a poly-N-substituted methacrylamide derivative or copolymers, a polyvinyl methyl ether and a partially acetylated polyvinyl alcohol.

12. The method of Claim 4, wherein said cell adhesive substance is selected from the group consisting of extracellular matrix components, gelatin, lectins, anchorage oligopeptides, adhesive proteins isolated from shellfish, positively charged polymers and their mixtures.

13. A cell cluster or sheet prepared by the method of Claim 4.

14. A prosthesis for repairing living tissue comprising the cell cluster or sheet of Claim 13.